# EUROPÄISCHE PATENTSCHRIFT

(11) **EP 0 931 793 B1**
(45) Veröffentlichungstag und Bekanntmachung des Hinweises auf die Patenterteilung: **17.03.2004**
(21) Anmeldenummer: 98124672.1
(22) Anmeldetag: 24.12.1998
(51) Int. Cl.: C07K 16/40, C07K 16/46, A61K 39/395, G01N 33/573, C07K 1/22

(54) **Monoklonaler Antikörper spezifisch für aktivierten Gerinnungsfaktor VII und seine Verwendung**
Monoclonal antibody specific for the activated coagulation factor VII (FVIIa) and its use
Anticorps monoclonal spécifique du facteur de coagulation VII activé et son utilisation

(30) Priorität: 22.01.1998 DE 19802139
(43) Veröffentlichungstag der Anmeldung: 28.07.1999
(73) Patentinhaber: Aventis Behring Gesellschaft mit beschränkter Haftung, 35002 Marburg (DE)
(72) Erfinder: Römisch, Jürgen, Dr., 35041 Marburg (DE); Lang, Wiegand, 35091 Cölbe (DE); Feussner, Annette, 35043 Marburg (DE); Röder, Joachim, 35041 Marburg (DE)

(56) Entgegenhaltungen:
- WO-A-97/26010
- US-A- 5 506 134
- PHILIPPOU H. ET AL.: "A novel specific immunoassay for plasma two-chain factor VIIa." BLOOD, NEW YORK, USA, Bd. 89, Nr. 3, 1. Februar 1997, Seiten 767-775, XP002101112
- PERSSON E. ET PETERSON L. C.: "Structurally and functionally distinct Ca2+ binding sites in the gamma-carboxyglutamic acid-containing domain of factor VIIa." EUROPEAN JOURNAL OF BIOCHEMISTRY, Bd. 234, Nr. 1, 15. November 1995, Seiten 293-300, XP002101115
- RÖMISCH J. ET AL.: "Characteristics and use of a monoclonal antibody specific for activated factor VII." BLOOD, NEW YORK, USA, Bd. 92, Nr. 10 suppl. 1 part. 1-2, 15. November 1998, Seite 99B XP002101113
- RÖMISCH J. ET AL.: "A monoclonal antibody specific for activated factor VII: Characteristics and use." ANNALS OF HEMATOLOGY, HEIDELBERG, DEUTSCHLAND, Bd. 76, Nr. suppl. 1, Februar 1998, Seite A50 XP002101114

## Beschreibung

Gegenstand der Erfindung ist ein monoklonaler Antikörper, der den aktivierten Gerinnungsfaktor VII (FVIIa) spezifisch bindet sowie seine Verwendung.

Die Blutgerinnung ist ein komplexes System, an dem Proteine in Form von Proteasen, Akzelleratoren und Inhibitoren beteiligt sind. Der überwiegende Anteil der Proteasen liegt in nicht-aktiviertem Zustand vor. Bei Auslösung der Gerinnung findet eine Umwandlung ihrer Pro-Formen in den aktivierten Zustand statt, woraus eine kaskadenförmige Aktivierung der Faktoren und somit eine Verstärkung der Reaktion resultiert. Grundsätzlich unterscheidet man die sog. endogenen und exogenen Gerinnungswege. Bei Gewebeverletzung wird die exogene Kaskade initiiert, indem Thromboplastin (TF=tissue factor) auf Zelloberflächen exponiert wird und den Gerinnungsfaktor VII (FVII) bzw. FVIIa bindet. Die Aktivierung des FVII findet autokatalytisch an TF statt oder über Proteasen wie Thrombin oder FXa. Der TF-VIIa-Komplex aktiviert FX zu FXa, wobei die anschließende Aktivierung von Prothrombin sich wiederum an Phospholipid-Oberflächen in Gegenwart von Kalzium vollzieht. Diese Reaktion wird durch FVa beschleunigt und führt über das entstandene Thrombin zur Bildung von Fibrin und dadurch zu einem Wundverschluß.

Obwohl die Gerinnungsfaktoren im Blut normalerweise in nicht-aktivierten Zustand vorliegen, wurden geringe Mengen FVIIa im Plasma gesunder Menschen detektiert. Dieser Mechanismus dient möglicherweise dazu, physiologisch sehr schnell auf kleinste Gewebeverletzungen reagieren zu können, wenn TF exponiert wird. Eine Korrelation von kursierenden, erhöhten FVIIa-Spiegeln könnte eine Rolle bei pathophysiologischen Reaktion spielen und diese auslösen, also zum Beispiel zu einem erhöhten Thromboserisiko führen.

Zur quantitativen Erfassung von FVII stehen derzeit Gerinnungstests zur Verfügung, die aufgrund ihrer Konzeption auch Spuren von FVIIa messen, also nicht zwischen FVII und FVIIa differenzieren können. Ein weitaus spezifischeres Testsystem zur Bestimmung von FVIIa wurde mit dem sog. rTF-FVIIa Test eingeführt. Verläßlich arbeitet dieses System besonders dann, wenn keine oder nur geringe Mengen an anderen aktivierten Faktoren wie FXa oder FIIa vorliegen. Bei höheren Konzentrationen aktivierter Faktoren können jedoch erhöhte Spiegel an FVIIa vorgetäuscht werden.

Neben der quantitativen Erfassung von Faktor VIIa in Körperflüssigkeiten, besonders im Plasma, ist die Bestimmung von FVII und/oder FVIIa enthaltenden Gerinnungsprodukten von großem Interesse. Beispielsweise werden sog. Prothrombinkomplexkonzentrate (PPSB) bei Mangelzuständen an den entsprechenden Faktoren (FII/FVII/FIX/FX etc.) Patienten verabreicht. Obwohl eine Erhöhung des Risikos thromboembolischer Komplikationen durch Anwesenheit von Spuren FVIIa nicht gezeigt werden konnte, werden möglichst geringe FVIIa-Gehalte in nicht-aktivierten PPSB-Konzentraten angestrebt. Die diesbezügliche Analytik ist also von erheblichem Interesse. Daneben werden für bestimmte Indikationen bereits aktivierte Komplexkonzentrate eingesetzt, wobei auch hier eine sorgfältige Quantifizierung der aktivierten Faktoren vorgenommen werden muß.

Neben dem rTF-FVIIa Assay, der die Aktivität des FVIIa mißt, ist ein Nachweissystem für FVIIa-Antigen wünschenswert. Der Erfindung lag also die Aufgabe zugrunde, eine Nachweismethode für FVIIa auf Antigenbasis bereitzustellen.

Gelöst wird diese Aufgabe durch einen monoklonalen Antikörper, der spezifisch den aktivierten Faktor VII bindet, den nicht aktivierten Faktor VII nicht bindet und einen mit Antithrombin III komplexierten, aktivierten Faktor VII nicht bindet.

Zu seiner Herstellung wurden Mäuse mit rekombinantem, aktiviertem Faktor VII immunisiert. Die Milzzellen der Maus wurden mit der murinen Myelom-Zellinie Sp2/0-Ag14 fusioniert. Als Fusionsreagenz wurde Polyetylenglykol 4.000 verwendet. Die Zellen wurden auf 24 Well-Kulturplatten verteilt. Als Medium wurde Dulbecco mod. Eagle's Medium mit 10% fötalem Kälberserum und HAT-Medium zur Selektion eingesetzt. Nach etwa 2 Wochen wurden die wachsenden Zellinien in die Vertiefungen einer 48 Well-Platte transferiert und kodiert. Von ca. 2.400 gewachsenen Zellinien wurde der Kulturüberstand genommen und mittels ELISA auf Anwesenheit von Maus-IgG getestet.

Mit Hilfe von immobilisiertem Faktor VII und aktiviertem Faktor VII (ELISA) wurden 392 Maus-IgG-positive Zellinien auf Spezifität geprüft. Von den getesteten Zellinien wurde 1 Zellinie mit der Kodenummer 1069/1373 als spezifisch für den aktivierten Faktor VII identifiziert. Sie ist bei der deutschen Sammlung für Mikroorganismen und Zellkulturen GmbH unter der Nr. DSM ACC 2332 hinterlegt worden. Die Spezifität des von dieser Zellinie gebildeten Antikörpers konnte im sog. BIAcore-System bestätigt werden. Der gereinigte monoklonale Antikörper ist vom Typ des IgG 1.

Der erfindungsgemäße monoklonale Antikörper wurde weiter charakterisiert, indem seine Fähigkeit der Inhibition von aktiviertem Faktor VII im Gerinnungstest geprüft wurde. Dabei stellte sich heraus, daß die Aktivität des aktivierten Faktor VII durch Inkubation mit mit dem monoklonalen Antikörper (mAb) 1069/1373 in einer von der Konzentration abhängigen Weise gehemmt wurde. SDS-PAGE vom Faktor VII und vom aktivierten Faktor VII, anschließender Transfer auf Nitrocellulose und Inkubation mit dem mAb 1069/1373 bestätigte, daß nur der aktivierte Faktor VII, nicht jedoch der Faktor VII selbst gebunden wurde und zu einer entsprechenden Markierung der Bande führte, wenn POD-gekoppelte Ziege-anti-Maus-Antikörper und ein entsprechendes Substrat zugesetzt wurden.

Ein weiteres Merkmal des mAb 1069/1373 ist, daß vor allem freier, aktivierter Faktor VII erkannt wird, d.h., daß keine Bindung von mit zum Beispiel Antithrombin III (ATIII)-komplexiertem, aktiviertem Faktor VII erfolgt. Folgendes Experiment verdeutlicht diese Eigenschaft:

Die in-vitro Herstellung solcher Komplexe gelingt durch Inkubation von aktiviertem Faktor VII mit einem Überschuß an Antithrombin III/Heparin bei 4°C für mehrere Stunden. Je nach Vollständigkeit der Komplexbildung von aktiviertem Faktor VII mit Antithrombin III ist der aktivierte Faktor VII im entsprechenden Aktivitätstest deutlich reduziert oder überhaupt nicht nachweisbar. In diesem Experiment wurde eine Abnahme der Aktivität des aktivierten Faktor VII von mehr als 90% gegenüber einer Kontrolle beobachtet. Ein entsprechend verändertes Signal wurde im Antigennachweissystem gefunden. Dies verdeutlicht, daß nur freier, aktivierter Faktor VII erkannt wird, jedoch nicht der Protease-Inhibitor-Komplex. Der mAb 1069/1373 eignet sich hervorragend zum qualitativen und quantitativen Nachweis von aktiviertem Faktor VII in Lösungen wie Körperflüssigkeiten oder in gelösten Gerinnungspräparaten oder Zwischenprodukten, die bei der Herstellung von Blutgerinnungsfaktoren anfallen. Die Etablierung eines entsprechenden ELISA-Tests ist im Beispiel 1 (siehe unten) beschrieben. Darüber hinaus ist der erfindungsgemäße monoklonale Antikörper 1069/1373 auch zum Nachweis der Bindung des aktiviertem Faktor VII an Zelloberflächen und Geweben geeignet. Zum Nachweis können bekannte Methoden eingesetzt werden wie die direkte Reaktion des mAb mit dem aktivierten Faktor VII oder auch ein indirekter Nachweis unter Verwendung eines gegen den mAb gerichteten Zweit-Antikörpers (Anti-Maus). Auch Antigen bindende Fragmente des erfindungsgemäßen monoklonalen Antikörpers, die den aktivierten Faktor VII bindende Regionen enthalten wie F(ab2) oder F(ab), können hierfür eingesetzt werden. Aufgrund seines inhibitorischen Potentiales kann neben dem mAb und dessen Fragmenten mit besonderem Vorteil ein entsprechender, humanisierter monoklonaler Antikörper zur prophylaktischen und/oder therapeutischen Anwendung kommen, insbesondere zur Verhinderung oder Behandlung thrombotischer Ereignisse. Ein derartiger humanisierter monoklonaler Antikörper umfaßt die den aktivierten Faktor VII bindenden hypervariablen Regionen des erfindungsgemäßen monoklonalen Antikörpers und die Framework-Regionen der variablen und konstanten Regionen der leichten und schweren Ketten eines humanen Antikörpers.

Außerdem kann der mAb auch zur Entfernung des aktivierten Faktor VII aus Lösungen verwendet werden. Beispielsweise kann man durch Koppelung an bekannte Matrices wie BrCN-Sepharose oder Protein A-Sepharose ein Affinitätsgel herstellen, auf der der erfindungsgemäße monoklonale Antikörper verankert ist. Leitet man dann die den aktivierten Faktor VII enthaltende Lösung über eine derartige Matrix, dann wird der aktivierte Faktor VII an ihr selektiv gebunden. Dadurch entsteht ein Gerinnungspräparat, das frei von aktiviertem Faktor VII ist.

Die Erfindung wird durch die folgenden Beispiele näher erläutert:

### Beispiel 1

### Verwendung des monoklonalen Antikörpers 1069/1373 zur Etablierung eines ELISAs für die quantitative Bestimmung des aktivierten Faktor VII

Für die quantitative Bestimmung des aktivierten Faktor VII wurde ein indirekter ELISA entwickelt: Der für den aktivierten Faktor VII spezifische monoklonale Antikörper 1069/1373 wurde durch Adhäsion an die Vertiefungen einer Mikrotiterplatte gebunden. Zur Sättigung nicht besetzter Bindungsstellen der Festphase wurde Rinder-Serumalbumin eingesetzt. Der aktivierte Faktor VII in den Proben bindet an den spezifischen Antikörper. Nicht gebundener, aktivierter Faktor VII wird durch Waschschritte entfemt. Als Zweit-Antikörper wird ein enzymmarkierter, für den Faktor VII spezifischer monoklonaler Antikörper verwendet. Der gebundene, aktivierte Faktor VII wird mit Hilfe einer von dem eingebrachten Enzym katalysierten Farbreaktion nachgewiesen.

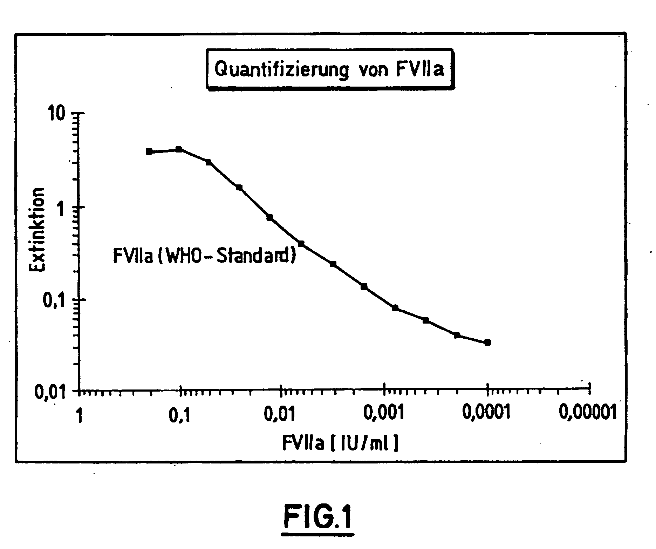

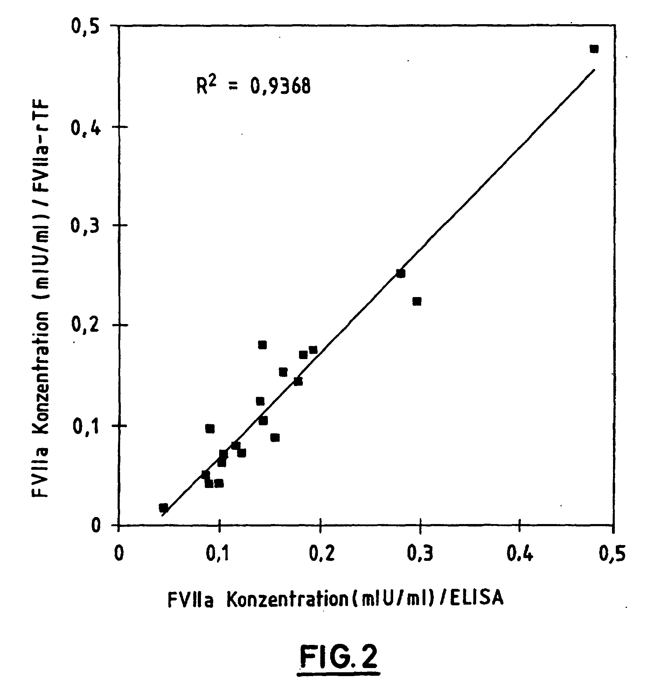

Die Gehalte an aktiviertem Faktor VII von Probenlösungen, beispielsweise Plasma oder daraus gewonnenen Produkten können mit Hilfe der in Fig. 1 dargestellten Standardkurve ermittelt werden. Die Spezifität dieses Tests für aktivierten Faktor VII und seine Verwendbarkeit wurde durch Zugabe von gereinigtem, aktivierten Faktor VII zu Faktor VII oder zu Plasma (nach Subtraktion von Baseline-Werten) und anschließender Quantifizierung von aktiviertem Faktor VII gezeigt.
Der etablierte ELISA wurde dazu verwendet, die Antigen-Konzentrationen von aktiviertem Faktor VII in Plasmen von 20 gesunden Spendern zu messen. Die ermittelten Konzentrationen von aktiviertem Faktor VII wurden mit Konzentrationen verglichen, die mit Hilfe des FVIIa-Aktivitätstests (Staclot® FVIIa-rTF der Firma Boehringer Mannheim/Stago) ermittelt wurden. Fig.2 zeigt eine sehr gute Korrelation der Ergebnisse beider Testsysteme.

Zudem wurde der ELISA dazu verwendet, eine aus der Plasmafraktionierung erhaltene Lösung, die eine Vielzahl von Proteinen enthielt, mit einer definierten Menge von aktiviertem Faktor VII zu versetzen und die FVIIa-Konzentrationen vor und nach der Zugabe zu quantifizieren. Tabelle 1 zeigt den Gehalt der Plasmafraktion (PF) an FVIIa vor Zugabe einer definierten Menge rFVIIa sowie die Analyse der zugebenen rFVIIa-Lösung und des Gemisches (PF+rFVIIa). Die ausgezeichnete Wiederfindung unterstreicht die Eignung des Tests auch in solch komplexen Proteinlösungen.

**Tabelle 1**

| **Probe** | **FVIIa Antigen-Konzentration (IU/ml)** | |
|---|---|---|
| | Mittelwert | Standardabweichung |
| rFVIIa | 5,02 | 0,19 |
| PF | 9,53 | 0,17 |
| PF + rFVIIa | 14,31 | 0,42 |

### Beispiel 2

### Verwendung des monoklonalen Antikörpers 106911373 zur Etablierung einer Immunaffinitätsmatrix zur Isolierung/Eliminierung von aktivierten Faktor VII

Gereinigter monoklonaler Antikörper 1069/1373 wurde an eine BrCN-Sepharose gebunden. Anschließend wurde ein Zwischenprodukt der Plasmafraktionierung, das unter anderem die Gerinnungsfaktoren FII, FVII, FIX und FX enthielt und mit einer bestimmten Menge gereinigten Faktor VIIa versetzt worden war, über diese mAb-Sepharose gepumpt. Die Lösungen und Säule waren in 50 mM Tris, 150 mM Natriumchlorid, 10 mM Calciumchlorid und pH 8,5 äquilibriert. Der Säulendurchlauf wurde aufgefangen. Nach Waschen des Säulenmaterials mit Äquilibrierungspuffer, der 0,5 Mol Natriumchlorid enthielt, erfolgte die Elution der Matrix mit einem Puffer aus 50 mM Natrium-Citrat, 100 mM Natriumchlorid bei pH 3,5.

Die Gehalte der obengenannten Gerinnungsfaktoren des Ausgangsmaterials, des Säulendurchlaufs und des Eluates wurden mit Hilfe von Gerinnungstests quantitativ erfaßt. Der aktivierte Faktor VII wurde mittels des FVIIa-rTF (Staclot®, Boehringer Mannheim, Stago) quantifiziert.

### Ergebnis:

Der aktivierte Faktor VII konnte aus der Ausgangslösung durch die Affinitätsmatrix eliminiert werden. Aktiver Faktor VII wurde im Eluat der Säule nachgewiesen. Die anderen getesteten Gerinnungsfaktoren wurden im Säulendurchlauf wiedergefunden. Dies zeigt, daß der aktivierte Faktor VII mit Hilfe des erfindungsgemäßen monoklonalen Antikörpers spezifisch isoliert und eliminiert werden kann. Im Eluat gewonnener, aktivierter Faktor VII liegt in aktiver Form vor und steht dann für andere Applikationen zur Verfügung.

## Patentansprüche

1. Monoklonaler Antikörper, **dadurch gekennzeichnet, daß** er spezifisch den aktivierten Faktor VII bindet, den nicht aktivierten Faktor VII nicht bindet, und einen mit Antithrombin III komplexierten, aktivierten Faktor VII nicht bindet.

2. Monoklonaler Antikörper nach Anspruch 1, **dadurch gekennzeichnet, daß** er von der Hybridoma-Zellinie DSM ACC 2332 gebildet wird.

3. Humanisierter monoklonaler Antikörper, **dadurch gekennzeichnet, daß** er die den aktivierten Faktor VII bindenden hypervariablen Regionen eines monoklonalen Antikörpers der Ansprüche 1 und 2 und die Frameworkregionen der variablen und konstanten Regionen der leichten und schweren Ketten eines humanen Antikörpers enthält.

4. Therapeutische Zubereitung, **dadurch gekennzeichnet, daß** sie den humanisierten monoklonalen Antikörper gemäß Anspruch 3 enthält.

5. Verfahren zur Entfernung von aktiviertem Faktor VII, **dadurch gekennzeichnet, daß** man eine den aktivierten Faktor VII enthaltende Lösung über eine Matrix leitet, auf der der monoklonale Antikörper der Ansprüche 1 bis 3 verankert ist.

6. Verwendung eines monoklonalen Antikörpers der Ansprüche 1 bis 3, **dadurch gekennzeichnet, daß** er zum qualitativen oder quantitativen Nachweis des aktivierten Faktor VII in Körperflüssigkeiten, Blutgerinnungspräparaten oder den Zwischenstufen ihrer Herstellung, auf Zelloberflächen oder in Geweben eingesetzt wird.

## Claims

1. A monoclonal antibody which specifically binds activated factor VII, does not bind non-activated factor VII, and does not bind an activated factor VII which is complexed with antithrombin III.

2. The monoclonal antibody as claimed in claim 1, which is formed by the hybridoma cell line DSM ACC 2332.

3. A humanized monoclonal antibody, which contains the activated factor VII-binding hypervariable regions of a monoclonal antibody as claimed in claims 1 and 2 and the framework regions of the variable and constant regions of the light and heavy chains of a human antibody.

4. A therapeutic preparation which comprises the humanized monoclonal antibody as claimed in claim 3.

5. A process for removing activated factor VII, which comprises passing an activated factor VII-containing solution through a matrix on which the monoclonal antibody as claimed in claims 1 to 3 is anchored.

6. The use of a monoclonal antibody as claimed in claims 1 to 3, wherein the antibody is used for qualitatively or quantitatively detecting activated factor VII in body fluids, blood coagulation preparations or the intermediate stages in the production of these preparations, on cell surfaces or in tissues.

## Revendications

1. Anticorps monoclonal, **caractérisé en ce qu'**il lie spécifiquement le facteur activé VII, **en ce qu'**il ne lie pas le facteur VII non activé et **en ce qu'**il ne lie pas un facteur VII activé complexifié avec de l'antithrombine m.

2. Anticorps monoclonal selon la revendication 1, **caractérisé en ce qu'**il est produit par la lignée cellulaire d'hybridome DSM ACC 2332.

3. Anticorps monoclonal humanisé, **caractérisé en ce qu'**il contient les régions hypervariables, liant le facteur VII activé, d'un anticorps monoclonal des revendications 1 et 2, et les régions cadre (framework) des régions variables et constantes des chaînes légères et lourdes d'un anticorps humain.

4. Préparation thérapeutique, **caractérisée en ce qu'**elle contient l'anticorps monoclonal humanisé selon la revendication 3.

5. Procédé d'élimination du facteur VII activé, **caractérisé en ce qu'**une solution contenant le facteur VII activé est conduite sur une matrice sur laquelle est ancré l'anticorps monoclonal des revendications 1 à 3.

6. Utilisation d'un anticorps monoclonal selon les revendications 1 à 3, **caractérisé en ce qu'**il est mis en oeuvre sur des surfaces cellulaires ou dans des tissus pour mettre en évidence qualitativement ou quantitativement le facteur VII activé dans des liquides corporels, des préparations de coagulation sanguine ou dans les étapes intermédiaires de leur préparation.
